# EUROPEAN PATENT SPECIFICATION

(11) **EP 2 716 252 B1**
(45) Date of publication and mention of the grant of the patent: **23.12.2015**
(21) Application number: 13187462.0
(22) Date of filing: 04.10.2013
(51) Int. Cl.: A61B 19/00

(54) **System and method for guiding the manual insertion of a needle into the body of a patient.**
System und Methode zum Führen einer manuellen Einbringung einer Nadel in den Körper eines Patienten.
Système et méthode pour guider une insertion manuelle d'une aiguille dans le corps d'un patient.

(30) Priority: 05.10.2012 IT BO20120547
(43) Date of publication of application: 09.04.2014
(73) Proprietor: Dall'Alba, Diego, Santorso (IT); Maris, Bogdan Mihai, Castelnuovo del Garda (IT); Fiorini, Paolo, Verona (IT)
(72) Inventor: Dall'Alba, Diego, Santorso (IT); Maris, Bogdan Mihai, Castelnuovo del Garda (IT); Fiorini, Paolo, Verona (IT)
(74) Representative: Boggio, Luigi

(56) References cited:
- US-A1- 2005 020 909
- US-A1- 2010 100 081
- US-B1- 7 302 288

## Description

The present invention relates to a system for guiding the manual insertion of a needle into the body of a patient during a percutaneous surgical procedure and a corresponding method for guiding the manual insertion of the needle.

In particular, the present invention is advantageously but not exclusively applied in percutaneous surgical procedures that make use of biopsy needles, to which the following description will explicitly refer without loss of generality.

Modern diagnostic and therapeutic surgical operations tend to make increasing use of percutaneous procedures. An example of a percutaneous procedure is a biopsy, which consists in inserting an elongate surgical instrument, typically a needle, into the body of the patient undergoing diagnosis so that the tip of the instrument reaches a target point inside the body to withdraw a sample of biological tissue (diagnosis) or to treat a portion of biological tissue of an internal organ (therapy).

These procedures require great ability on the part of the doctor who manipulates the needle, since it is undoubtedly easy to initially position the tip of the needle in the entry point on the skin of the body but it is difficult to correctly manipulate the needle to reach the target point without being able to see the inside of the body. The use of image acquisition equipment is known, for example equipment for CAT, MRI or ultrasound scans, to acquire images of the inside of the body around the target point in order to verify correct insertion of the needle.

When the percutaneous procedure is guided by means of computer tomography, a scan is performed before inserting the needle, to identify the best positions for the entry point and the target point, and therefore to determine the inclination of and depth at which to insert the needle. The needle insertion operation is performed "blind" without any actual real-time feedback on the insertion. A variable number of scans are then performed to evaluate correct insertion of the needle, i.e. to evaluate the correct position of the tip and the correct orientation of the needle axis, and to verify that the passage of the needle does not damage structures at risk. Each scan requires insertion of the needle to be interrupted, consequently prolonging the duration of the operation and discomfort of the patient, who is usually given a local anaesthetic. The success of insertion of the needle depends to a great extent on the ability of the radiologist who controls the image acquisition equipment, and on that of the doctor who manipulates the needle. As the number of scans necessary for verifying that the target point has been correctly reached increases, the dose of ionizing radiation to which the patient is exposed increases, with all related risks and problems.

Analogous considerations apply when the percutaneous procedure is guided by magnetic resonance imaging. In this case, the patient is not subjected to ionizing radiation but to an intense magnetic field which makes the procedure very complex to perform due to the interferences with metallic and electronic objects. Furthermore, the magnetic resonance equipment is very bulky, making it more complicated to manipulate the needle, given the reduced access to the patient and the longer scanning times, with consequent increase in the time required for performance of the percutaneous procedure.

When an ultrasound scan is used, it is possible to monitor insertion of the needle in real time as there are no problems connected with radiation doses or magnetic fields. However, ultrasound scanning requires good coordination between manipulation of the needle and acquisition of the images, since it is difficult to keep the needle in the plane of the ultrasound probe. Furthermore, the quality of the ultrasound images is decidedly inferior to that of the images obtained by computed tomography or magnetic resonance imaging, to the extent that even expert personnel with many years of training have considerable difficulty in accurately locating the structures of interest.

The object of the present invention is to provide a system and a method for guiding the manual insertion of a needle into the body of a patient, which are free from the drawbacks described above and, at the same time, are easy and inexpensive to implement.

According to the present invention, a system and a method are provided for guiding the manual insertion of a needle into the body of a patient during a percutaneous surgical procedure, as defined in the attached claims 1 and 8. US Patent Application US 2010/0100081 discloses the preamble of claim 1.

For a better understanding of the present invention, a preferred embodiment is now described, purely by way of nonlimiting example and with reference to the accompanying drawings, in which:
- figure 1 illustrates, schematically, the guiding system for manual insertion of a needle into the body of a patient, produced according to the present invention;
- figure 2 illustrates, according to a perspective view, a guiding device of the guiding system of figure 1, said guiding device being mounted on the needle;
- figure 3 illustrates a calibration system to allow calibration of the guiding device of figure 2;
- figures 4 and 5 illustrate, schematically, the way in which graphical navigation information is determined, that graphical navigation information being displayed by means of a graphical display forming part of the guiding device of figure 2;
- figure 6 illustrates a state diagram relative to operation of the guiding system of figure 1; and
- figures 7 to 9 illustrate respective examples of graphical navigation information displayed by means of the graphical display of the guiding device of figure 2.

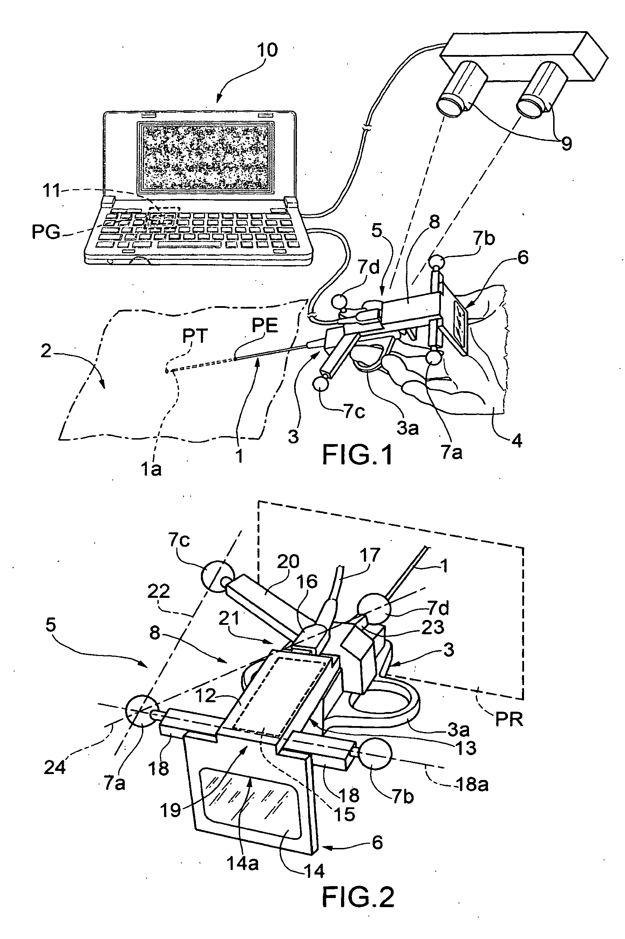

In figure 1, the number 1 generically indicates a needle for surgical procedures suited to be inserted into the body of a patient, the latter shown only by a patch of skin and indicated by 2. The needle 1 is illustrated partially inserted into the body 2, therefore with its tip 1a inside the body 2. The needle 1 is of known type, for example a biopsy needle comprising a head 3 with a pair of eyelets 3a, only one of which is visible in figure 1, to facilitate gripping of the hand 4 of the doctor who performs manual insertion of the needle 1 into the body 2.

With reference to figure 1, the guiding system invented comprises, firstly, a guiding device 5 mounted rigidly on the head 3 of the needle 1. The guiding device 5 comprises a graphical display 6, at least three markers 7a-7d consisting of respective elements able to reflect the infrared radiation and a supporting frame 8 to sustain the graphical display 6 and the markers 7a-7d and allow the guiding device 5 to be mounted on the needle 1 with the markers 7a-7d arranged in a certain geometrical pattern with respect to the tip 1a of the needle 1. Each marker 7a-7d is in the form of a sphere.

The guiding system furthermore comprises at least two infrared video cameras 9 arranged at a reciprocal distance to acquire, from at least two different observation points, images of the guiding device 5 during the surgical procedure. Advantageously, there are four markers 7a-7d, as in the example of figure 1, to increase the probability of always identifying at least three of them on the images acquired.

The guiding system also comprises a processing device 10, for example a laptop computer, which is interfaced with the video cameras 9 and with the graphical display 6. The processing device 10 comprises a non-volatile memory 11, for example the hard disk of the laptop computer, to store the positions, in a system of coordinates which is fixed with respect to the operating table, of an entry point PE of the needle on the body 2 and a target point PT inside the body 2 which has to be reached by the tip 1a of the needle 1. The entry point PE and the target point PT are acquired manually by the doctor, before insertion of the needle 1, by means of an ultrasound instrument (not illustrated) interfaced with the processing device 10.

The memory 11 furthermore stores a program for processor PG which implements, when run on the calculation unit of the processor device 10, a series of processing steps starting from the images acquired and taking account of the positions of the entry point PE and the target point PT to generate graphical navigation information for display via the graphical display 6, as will be explained in detail further on.

The video cameras 9 and first portions of software code of the processor program constitute a tracking system of known type. The first portions of software code implement processing steps necessary to determine the position of the markers 7a-7d, in the fixed coordinates system, on the basis of the images acquired by the video cameras 9.

Advantageously, there are more than two video cameras 9 so that it is always possible to accurately identify all the markers 7a-7d, whatever their spatial position. In one preferred embodiment not illustrated, there are at least ten video cameras 9 and they are mounted on a system of beams arranged around the operating table.

With reference to figure 2, the supporting frame 8 comprises a box-shaped element 12 which can be mounted on the head 3 of the needle 1 with a wall 13 of the box-shaped element 12 substantially parallel to the needle 1 and in contact with the head 3 of the needle 1, via coupling means, for example screws or male-female couplings. The graphic display 6 comprises a screen 14 fixed to the box-shaped element 12 so that it is perpendicular to the wall 13. In other words, the supporting frame 8 allows the guiding device 5 to be mounted on the needle 1 with the screen 14 parallel to a reference surface PR perpendicular to the axis of the needle 1.

The screen 14 is, for example, of the liquid crystal black and white type with a resolution of approximately 100x100 pixels and has an LED backlighting. This solution allows maximum reduction of power consumption. The control electronics 15 of the graphic display 6 are housed inside the box-shaped element 12. The control electronics 15 are electrically powered and interfaced to the processor device 10 by means of a USB socket 16 provided on an aperture (not visible) of the box-shaped element 12. The number 17 indicates the USB cable connected to the USB socket 16.

The supporting frame 8 comprises a pair of arms 18 which project from opposite sides of a front portion 19 of the box-shaped element 12, along the same axis 18a parallel to a longer side 14a of the screen 14 to support, at the respective free ends, two respective markers 7a and 7b.

The supporting frame 8 comprises, furthermore, another arm 20 which projects from a rear portion 21 of the box-shaped element 12 to support a third marker 7c, aligned with the marker 7a along an axis 22 which lies, together with the axis 18a, on a plane P2 (not illustrated in figure 1) that intersects obliquely the reference plane PR. The supporting frame 8 comprises, lastly, a further arm 23 which projects from the rear portion 21 to support the fourth marker 7d in the figure 2, aligned with the marker 7a along an axis 24 which lies, together with the axis 18a, on a plane P3 (not illustrated in figure 1) that intersects obliquely the reference plane PR and the plane P2.

The guiding system comprises a calibration device, which is used in combination with the video cameras 9 and with the processor device 10 to calibrate the guiding device 5 after the latter has been mounted on the needle 1, i.e. to determine the geometrical arrangement of the markers 7a-7d with respect to the tip 1a of the needle, and in particular to determine a geometrical transformation matrix M which allows passage from the position coordinates of the markers 7 to the position coordinates of the tip 1a and to the direction of the axis of the needle 1.

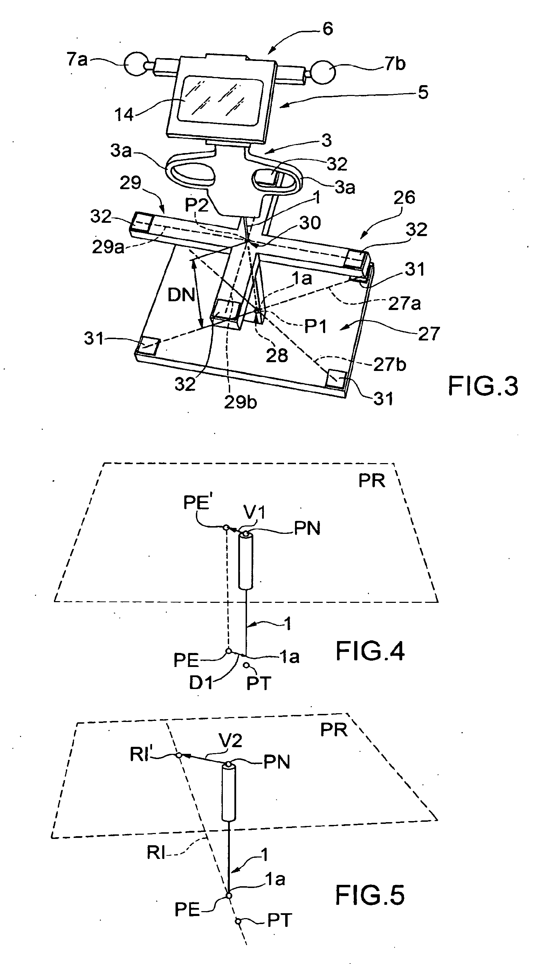

With reference to figure 3, the calibration system comprises a supporting frame 26 for the needle, which comprises a rectangular base 27 presenting a non-through hole 28 to receive the tip 1a of the needle 1 and a plate 29 rigidly connected to the base 27 at a certain distance DN from the base 27 and presenting a through hole 30 suited to be crossed by the stem of the needle 1 when the tip 1a engages the hole 28. In the example of figure 3, the plate 29 has the shape of a cross. The calibration system comprises, furthermore, a first group of four markers 31 applied to the base 27 so as to define the ends of two segments, for example the two diagonals 27a and 27b of the base 27, which intersect at point P1 situated in the hole 28, and a second group of markers 32 applied to the plate 29 so as to define the ends of another two segments, for example the cross-shaped axes 29a and 29b of the plate 29, which intersect at a point P2 situated in the hole 30. The markers 31 and 32 have the same behaviour as the markers 7a-7d, but they have a different shape, for example flat polygonal.

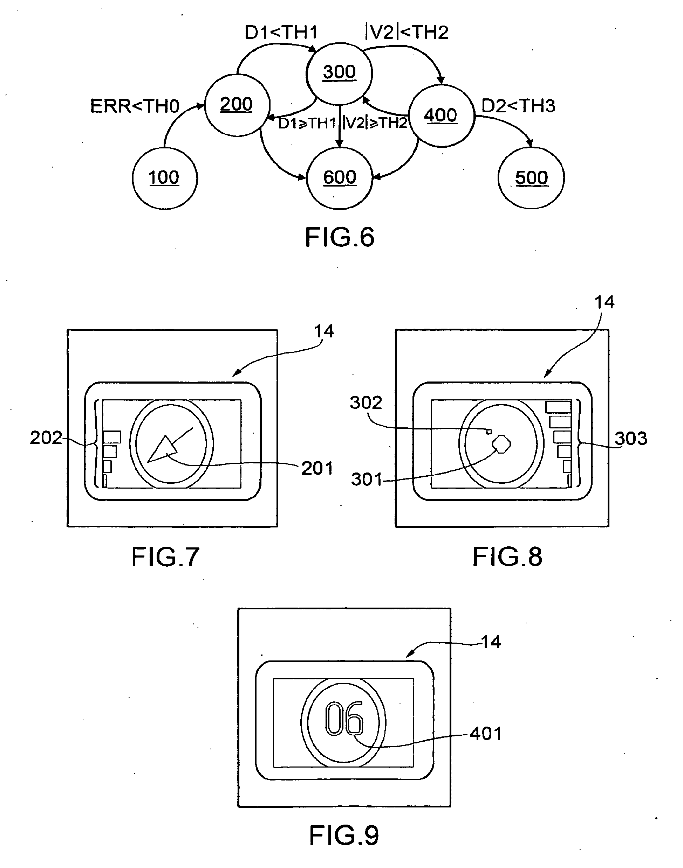

The program PG implements, in general, the state diagram of figure 6. In each state, the program PG implements, when run on the calculation unit of the processing device 10, a respective sequence of processing steps. The state diagram begins with an initial state 100 relative to a calibration step.

To perform the calibration, the needle 1 must be positioned, with the guiding device 5 mounted above, in the holes 28 and 30 of the supporting frame 26, as illustrated in figure 3. The calibration phase, which is performed automatically when the doctor gives the processing device 10 an appropriate command, comprises the following steps:
- acquire, by means of the video cameras 9, one or more images of the unit consisting of the guiding device 5 mounted on the needle 1;
- determine the position of all the markers 7a-7d, 31 and 32 starting from the images acquired; and
- calculate the position of the points P1 and P2 as a function of the position of the markers 31 and 32; and
- determine the geometrical transformation matrix M among the markers 7a-7d and the tip 1a and axis of the needle 1 as a function of the position of the markers 7a-7d and the points P1 and P2.

The calibration terminates after acquiring a number of images such that the transformation matrix M provides the position of the tip 1a of the needle 1 with an error margin ERR lower than a pre-set threshold TH0, for example with value equal to 1 mm.

The geometrical transformation matrix M assumes that the straight line passing through the points P1-P2 coincides with the axis of the needle 1. Said matrix M is stored in the memory 11 for use in the other operating states.

At this point, the guiding system is ready to be used during a surgical procedure for guiding manual insertion of the needle 1 into the body of a patient. In particular, the program PG, quitting the initial state 100, goes to the state for location of the entry point 200 and, when certain conditions occur, passes to the state in which it searches for the direction of insertion 300 and subsequently to the state of insertion 400. In all the states 200, 300 and 400, video images of the guiding device 5 during the surgical procedure are acquired by means of the video cameras 9 according to a pre-defined frame-rate, the positions of the markers 7a-7d are determined in real time on the basis of the images acquired, and the current position of the needle is calculated, i.e. the current position of the tip 1a and the current direction (orientation) of the axis of the needle 1, transforming the position coordinates of the markers 7a-7d by means of the geometrical transformation matrix M.

In the state 200 graphical information is generated and displayed on the screen 14 suited to guide the doctor in correct positioning of the tip 1a of the needle 1 on the entry point PE. When the program PG is in state 200, the doctor must move the guiding device 5 preferably with the screen 14 substantially parallel to the surface of the body 2 to best interpret the graphical information displayed.

With reference to figure 4, which schematically shows the situation in which the tip 1a of the needle 1 is still far from the entry point PE, in state 200 direction and sense of a vector V1 are calculated, which consists of the relative position vector of a point PE' defined by the orthogonal projection on the reference plane PR of the entry point PE, with respect to another point PN constituted by the current position of the needle 1 on the reference plane PR, and a first distance D1 is calculated between the current position of the tip 1a of the needle 1 and the position of the entry point PE. It should be noted that, since the axis of the needle 1 is perpendicular to the reference plane PR, the current position of the needle 1 on the reference plane PR, i.e. the point PN, coincides with the orthogonal projection on the plane PR of the tip 1a of the needle 1. The graphical information to be displayed is generated as a function of the vector V1 and the distance D1.

In the embodiment example shown by figure 7, the graphical information displayed in state 200 comprises a mobile arrow 201 which replicates exactly direction and sense of the vector V1 and a bar graph 202 which provides an indication of the distance D1. For example, as the distance D1 is reduced, the number of bars (i.e. the height of the bar graph) displayed diminishes. To best interpret this graphical information, the doctor must position the guiding device 5 with the screen 14 substantially parallel to the surface of the body 2. The graphical information 201, 202 is displayed until the distance D1 is greater than or equal to a first threshold TH1 with value, for example, of less than 3 mm.

When the distance D1 is less than the threshold TH1, the tip 1a of the needle 1 is considered on the entry point PE and the program PG goes to state 300, in which new graphical information is generated and displayed, suited to guide the doctor in searching for the correct insertion direction (orientation of the needle). In state 300, to best interpret the relative graphical information, the doctor should keep the tip 1a of the needle 1 still, while moving the needle 1, by oscillation, in search of the insertion direction.

With reference to figure 5, which schematically shows the situation in which the tip 1a of the needle 1 is at a standstill on the entry point PE, in state 300 modulus, direction and sense of a vector V2 are calculated, which consists of the relative position vector of a point RI' defined by the intersection between the reference plane PR and a straight line RI passing through the entry point PE and the target point PT, with respect to the point PN constituted by the current position of the needle 1 on the reference plane PR. Note that the straight line RI defines the trajectory of correct insertion. The graphical information to be displayed is generated as a function of the vector V2.

In the implementation example shown by figure 8, the graphical information displayed in state 300 comprises a small circle 301 centred in the screen 14, a mobile dot 302, the position of which with respect to the circle 301 is a function of the direction and sense of the vector V2, and a bar graph 303 which provides an indication of the modulus of the vector V2. The graphical information 301-303 is displayed until the modulus of the vector V2 is greater than or equal to a second threshold TH2 with value depending on the accuracy to be obtained and the distance between entry point PE and target point PT.

When the modulus of the vector V2 goes below the threshold TH2, the axis of the needle 1 is considered oriented according to the required direction of insertion and the program PG goes to state 400, in which new graphical information is generated and displayed suited to guiding the doctor to the target point PT. In state 400, to best interpret the relative graphical information, the doctor should insert the needle 1 in the body 2 keeping the needle 1 oriented according to the direction found.

In state 400 the distance D2 between the current position of the tip 1a of the needle 1 and the target point PT is calculated. The graphical information displayed in state 400 is indicative of the distance D2. In the example of implementation shown by figure 9, the graphical information displayed in state 400 comprises a percentage number 401 which represents the distance D2 as a percentage of the distance between the entry point PE and the target point PT. The percentage number 401 is displayed until the distance D2 is greater than or equal to a third threshold TH3 with value, for example, of less than 5 mm.

When the distance D2 is less than the threshold TH3, the tip 1a of the needle 1 is considered on the target point PT and the program PG goes to the state of target reached 500 (figure 6), in which new graphical information is generated and displayed indicating that the tip 1a has reached the target and that therefore the doctor can perform the next step in the surgical procedure, for example ablation of tissue or sampling of tissue.

The program PG implements, furthermore, a state of shutdown 600 which is reached from one of the states 200, 300 or 400 when a malfunction occurs or the calculations give unexpected results.

The main advantage of the guiding system described above is that it does not require, during insertion of the needle in the body of the patient, the aid of equipment for CAT or MRI scanning, and therefore it avoids all the relative drawbacks, such as the administration of doses of ionizing radiation or magnetic fields. In fact, the guiding system of the invention only requires an initial scan to identify the entry point PE and the target point PT and a final scan to verify that the target point PT has been reached, whereas the guided procedures by means of CAT or MRI scan require, on average, approximately twenty scans. Furthermore, the guiding system of the present invention allows the target point PE to be reached with extreme precision and therefore, by reducing the probability of having to repeat insertion of the needle, allows the entire surgical procedure to be performed in a shorter time. Lastly, the guiding system of the invention integrates perfectly with the use of an ultrasound instrument: in fact, use of the latter is not harmful and therefore allows insertion of the needle to be monitored in real time, the poor quality of the images produced being compensated for by the precision of the guiding system of the present invention.

## Claims

1. A system for guiding the manual insertion of a needle into the body of a patient during a percutaneous surgical procedure, the system comprising: a guiding device (5) which comprises a graphical display (6), a plurality of markers (7a-7d) suited to reflect infrared light, and support means (8) to allow the guiding device (5) to be mounted on the needle (1) with the markers (7a-7d) arranged according to a certain geometrical pattern with respect to the tip (1a) of the needle (1) and with the screen (14) of the graphical display (6) parallel to a first plane (PR) perpendicular to the axis of the needle (1); infrared image acquisition means (9) to acquire images of the guiding device (5) during the surgical procedure; memory means (11) for storing the positions, in a coordinate system that is fixed with respect to the operating table, of an entry point (PE) of the needle (1) on the body (2) and of a target point (PT) inside the body (2) that has to be reached by the tip (1a) of the needle (1); and processing means (10), which are interfaced with said image acquisition means (9) and with said graphical display (6) and are configured to determine in real time the position of the markers (7a-7d) in said coordinate system based on the images acquired and the current position of the needle (1) as a function of the positions of the markers (7a-7d) and of said geometrical pattern, and to control the graphical display (6) so that it displays graphical navigation information, which is a function of the current position of the needle (1) and of the positions of the entry and target points (PE, PT); the system being **characterised in that** said processing means (10) are configured to calculate direction and sense of a first vector (V1) consisting of the relative position vector, with respect to the current position of the needle (1) on said first plane (PR), of the orthogonal projection on said first plane (PR) of the entry point (PE), and calculate a first distance (D1) between the current position of the tip (1a) of the needle (1) and the position of the entry point (PE); said graphical navigation information comprising first graphical information (201, 202) indicative of said direction and sense of the first vector (V1) and of said first distance (D1).

2. A system according to claim 1, wherein said plurality of markers (7a-7d) comprise a first and a second marker (7a, 7b) aligned along a first axis (18a), which is parallel to a side (14a) of the screen (14) of said graphical display (6).

3. A system according to claim 2, wherein said plurality of markers (7a-7d) comprise a third marker (7c) aligned with the first marker (7a) along a second axis (22) which lies, together with said first axis (18a), on a second plane (P2) which obliquely intersects the first plane (PR).

4. A system according to claim 3, wherein said plurality of markers (7a-7d) comprise a fourth marker (7d) aligned with the first marker (7a) along a third axis (24) which lies, together with the first axis (18a), on a third plane (P3) which obliquely intersects both the first and the second plane (PR, P).

5. A system according to any of the claims from 1 to 4, wherein said processing means (10) are configured to calculate, when said first distance (D1) is less than a first threshold (TH1), modulus, direction and sense of a second vector (V2), which consists of the relative position vector, with respect to the current position of the needle (1) on said first plane (PR), of the intersection between said first plane (PR) and a straight line (RI) passing through the entry point (PE) and the target point (PT); said graphical navigation information comprising second graphical information (301-303) indicative of said modulus, direction and sense of the second vector (V2).

6. A system according to claim 5, wherein said processing means (10) are configured to calculate, when the modulus of said second vector (V2) is lower than a second threshold (TH2), a second distance (D2) between the current position of the tip (1a) of the needle (1) and the target point (PT); said graphical navigation information comprising third graphical information (401) indicative of said second distance (D2).

7. A system according to any of the claims from 1 to 6 and comprising calibration means (26, 31, 32) having further support means (26) for the needle (1), which present a first non-through hole (28) for receiving the tip (1a) of the needle (1) and a second through hole (30) arranged at a certain distance (DN) from the first hole (28) and suited to be run through by the stem of the needle (1) when the tip (1a) engages the first hole (28), two groups of four further markers (31, 32) each, which are suited to reflect infrared light and are applied onto the further support means (26) so as to define, the first group (31), the ends of two first segments (27a, 27b) that intersect in the first hole (28) and, the second group (32), the ends of two second segments (29a, 29b) that intersect in the second hole (30); said processing means (10) being configured to determine the positions of all the markers (7a-7d, 31, 32) based on at least one image, acquired by said image acquisition means (9), of the guiding device (5) mounted on the needle (1) with the latter being arranged in said first and second hole (28, 30), and to determine said geometrical pattern based on said positions of all the markers (7a-7d, 31, 32).

8. A method for guiding the manual insertion of a needle into the body of a patient during a percutaneous surgical procedure, the method comprising:
- acquiring the positions, in a coordinate system that is fixed with respect to the operating table, of an entry point (PE) of the needle (1) on said body (2) and of a target point (PT) inside said body (2) that has to be reached by the tip (1a) of the needle (1);
- mounting, on the needle (1), a graphical display (6) with its screen (14) arranged parallel to a first plane (PR), which is perpendicular to the axis of the needle (1), and a plurality of markers (7a-7d) suited to reflect the infrared radiation and arranged according to a certain geometrical pattern with respect to the tip (1a) of the needle (1);
- acquiring images of the markers (7a-7d) above the operating table during the surgical procedure by means of infrared image acquisition means (9);
- determining in real time the position, in said coordinate system, of the markers (7a-7d) based on the images acquired and the current position of the needle (1) as a function of the positions of the markers (7a-7d) and of said geometrical pattern;
- generating graphical navigation information as a function of the current position of the needle (1) and of the positions of the entry and target points (PE, PT);
- displaying, by means of said graphical display (6), the graphical navigation information;
the method being **characterised in that** the generation of graphical navigation information comprises:
- calculating direction and sense of a first vector (V1), which consists of the relative position vector, with respect to the current position of the needle (1) on said first plane (PR), of the orthogonal projection of the entry point (PE) onto said first plane (PR);
- calculating a first distance (D1) between the current position of the tip (1a) of the needle (1) and the position of the entry point (PE); and
- generating first graphical information (201, 202), which is indicative of said direction and sense of the first vector (V1) and of said first distance (D1).

9. A method according to claim 8, wherein displaying the graphical navigation information comprises:
- displaying said first graphical information (201, 202) at least until the first distance (D1) is greater than or equal to a first threshold (TH1).

10. A method according to claim 8 or 9, wherein generating graphical navigation information comprises, when the first distance (D1) is less than a first threshold (TH1):
- calculating modulus, direction and sense of a second vector (V2), which consists of the relative position vector, with respect to the current position of the needle (1) on said first plane (PR), of the intersection between said first plane (PR) and a straight line (RI) passing through the entry point (PE) and the target point (PT); and
- generating second graphical information (301-303), which is indicative of said modulus, direction and sense of the second vector (V2).

11. A method according to claim 10, wherein displaying the graphical navigation information comprises:
- displaying said second graphical information (301-303) at least until the modulus of the second vector (V2) is higher than, or equal to, a second threshold (TH2).

12. A method according to claim 10 or 11, wherein generating graphical navigation information comprises, when the modulus of the second vector (V2) is lower than the second threshold (TH2) :
- calculating a second distance (D2) between the current position of the tip (1a) of the needle (1) and the position of the target point (PT); and
- generating third graphical information (401), which is indicative of the second distance (D2);
displaying the graphical navigation information comprising:
- displaying the third graphical information until the second distance (D2) is higher than, or equal to, a third threshold (TH3).

## Patentansprüche

1. System zum Führen des manuellen Einstechens einer Nadel in den Körper eines Patienten während eines perkutanen chirurgischen Eingriffs, wobei das System aufweist: Eine Führungsvorrichtung (5), die ein Grafikdisplay (6) aufweist, eine Mehrzahl von zur Reflektion von Infrarotlicht geeigneten Markern (7a-7d), und Befestigungsmittel (8), die erlauben, die Führungsvorrichtung (5) an der Nadel (1) zu befestigen, wobei die Marker (7a-7d) entsprechend einem bestimmten geometrischen Muster in Bezug auf die Spitze (1a) der Nadel angeordnet sind und der Bildschirm (14) des Grafikdisplays (6) parallel zu einer ersten Ebene (PR) im rechten Winkel zu der Achse der Nadel (1) angeordnet ist; Infrarotbilderfassungsmittel (9) zur Erfassung von Bildern der Führungsvorrichtung (5) während des chirurgischen Eingriffs; Speichermittel (11) zum Speichern in einem in Bezug auf den Operationstisch festen Koordinatensystem der Positionen eines Einstichpunktes (PE) der Nadel (1) in den Körper (2) und eines Zielpunktes (PT) innerhalb des Körpers (2), der von der Spitze (1 a) der Nadel (1) erreicht werden soll; und Verarbeitungsmittel (10), die an die Bilderfassungsmittel (9) und das Grafikdisplay (6) gekoppelt sind und zur Bestimmung der Position der Marker (7a-7d) in dem Koordinatensystem auf der Grundlage der erfassten Bilder und der aktuellen Position der Nadel (1) als eine Funktion der Positionen der Marker (7a-7d) und des geometrischen Musters in Echtzeit ausgebildet sind, und zur Kontrolle des Grafikdisplays (6) ausgebildet sind, so dass dieses grafische Navigationsinformation anzeigt, die eine Funktion der aktuellen Position der Nadel (1) und der Positionen der Einstichstelle und des Zielpunktes (PE, PT) ist; wobei das System **dadurch gekennzeichnet ist, dass** die Verarbeitungsmittel (10) zur Berechnung der Lage (*direction*) und Richtung (*sense*) eines ersten Vektors (V1) bestehend aus dem relativen Ortsvektor, in Bezug auf die aktuelle Position der Nadel (1) in der ersten Ebene (PR), der orthogonalen Projektion auf die erste Ebene (PR) der Einstichstelle (PE), und zur Berechnung eines ersten Abstandes (D1) zwischen der aktuellen Position der Spitze (1a) der Nadel (1) und der Position der Einstichstelle (PE) ausgebildet sind; wobei die grafische Navigationsinformation erste grafische Information (201, 202) aufweist, die auf die Lage und Richtung des ersten Vektors (V1) und des ersten Abstandes (D1) hinweist.

2. System nach Anspruch 1, wobei die Mehrzahl der Marker (7a-7d) einen ersten und einen zweiten Marker (7a, 7b) aufweist, die entlang einer ersten Achse (18a) angeordnet sind, die parallel zu einer Seite (14a) des Bildschirms (14) des Grafikdisplays (6) ist.

3. System nach Anspruch 2, wobei die Mehrzahl der Marker (7a-7d) einen dritten Marker (7c) aufweist, der auf den ersten Marker (7a) entlang einer zweiten Achse (22) ausgerichtet ist, die zusammen mit der ersten Achse (18a) in einer zweiten Ebene (P2) liegt, die die erste Ebene (PR) schräg schneidet.

4. System nach Anspruch 3, wobei die Mehrzahl der Marker (7a-7d) einen vierten Marker (7d) aufweist, der auf den ersten Marker (7a) entlang einer dritten Achse (24) ausgerichtet ist, die zusammen mit der ersten Achse (18a) in einer dritten Ebene (P3) liegt, die die erste und zweite Ebene (PR, P) schräg schneiden.

5. System nach einem der Ansprüche 1 bis 4, wobei die Verarbeitungsmittel (10) zur Berechnung, wenn der erste Abstand (D1) kleiner als ein erster Grenzwert (TH1) ist, des Betrags, der Lage und Richtung eines zweiten Vektors (V2) ausgebildet sind, der besteht aus dem relativen Ortsvektor, in Bezug auf die aktuelle Position der Nadel (1) in der ersten Ebene (PR), des Schnittpunktes der ersten Ebene (PR) und einer sich durch die Einstichstelle (PE) und den Zielpunkt (PT) erstreckenden geraden Linie; wobei die grafische Navigationsinformation zweite grafische Information (301 bis 303) aufweist, die auf den Betrag, die Lage und Richtung des zweiten Vektors (V2) hinweist.

6. System nach Anspruch 5, wobei die Verarbeitungsmittel (10) zur Berechnung, wenn der Betrag des zweiten Vektors (V2) kleiner als ein zweiter Grenzwert (TH2) ist, eines zweiten Abstandes (D2) zwischen der aktuellen Position der Spitze (1a) der Nadel (1) und dem Zielpunkt (PT) ausgebildet sind; wobei die grafische Navigationsinformation dritte grafische Information (401) aufweist, die auf den zweiten Abstand (D2) hinweist.

7. System nach einem der Ansprüche 1 bis 6 und aufweisend Kalibriermittel (26, 31, 32), die weitere Befestigungsmittel (26) für die Nadel (1) haben, die präsentieren eine erste nicht durchgehende Bohrung (28) zur Aufnahme der Spitze (1a) der Nadel (1) und eine zweite durchgehende Bohrung (30), die in einem bestimmten Abstand (DN) von der ersten Bohrung (28) angeordnet ist und geeignet ist, von der Stange der Nadel (1) durchlaufen zu werden, wenn die Spitze (1a) in die erste Bohrung (28) eingreift, zwei Gruppen von vier weiteren Markern (31, 32), die jeweils zur Reflektion von Infrarotlicht geeignet sind und auf die weiteren Befestigungsmittel (26) derart aufgelegt sind, dass diese die erste Gruppe (31), die Enden von zwei ersten Segmenten (27a, 27b), die sich in der ersten Bohrung (28) schneiden, und die zweite Gruppe (32) definieren, die Enden von zwei zweiten Segmente (29a, 29b), die sich in der zweiten Bohrung (30) schneiden; wobei die Verarbeitungsmittel (10) zur Bestimmung der Positionen sämtlicher Marker (7a-7d, 31, 32) auf der Basis mindestens eines von den Bilderfassungsmitteln erfassten Bildes der Führungsvorrichtung (5), die an der Nadel (1) mit der letzteren in der ersten und zweiten Bohrung (28, 30) angeordnet befestigt ist, und zur Bestimmung des geometrischen Musters auf der Basis der Positionen sämtlicher Marker (7a-7d, 31, 31) ausgebildet sind.

8. Verfahren zum Führen des manuellen Einstechens einer Nadel in den Körper eines Patienten während eines perkutanen chirurgischen Eingriffs, wobei das Verfahren aufweist:
- Erfassen in einem in Bezug auf den Operationstisch festen Koordinatensystemen der Positionen eines Einstichpunktes (PE) der Nadel (1) in den Körper (2) und eines Zielpunktes (PT) innerhalb des Körpers (2), der mit der Spitze (1 a) der Nadel erreicht werden soll;
- Befestigen eines Grafikdisplays (6) an der Nadel mit dessen Bildschirm (14) in einer parallelen Anordnung zu der ersten Ebene (PR), die rechtwinklig zu der Achse der Nadel (1) ist, und einer Vielzahl von Markern (7a-7d), die zur Reflektion von Infrarotstrahlung geeignet sind und entsprechend einem bestimmten geometrischen Muster in Bezug auf die Spitze (1a) der Nadel (1) angeordnet sind;
- Erfassen Bilder der Marker (7a-7d) oberhalb des Operationstisches während des chirurgischen Eingriffs mittels Infrarotbilderfassungsmittel (9);
- Bestimmen in Echtzeit in dem Koordinatensystem der Position der Marker (7a-7d) auf der Basis der erfassten Bilder und der aktuellen Position der Nadel (1) als eine Funktion der Positionen der Marker (7a-7d) und des geometrischen Musters;
- Generieren von grafischer Navigationsinformation als eine Funktion der aktuellen Position der Nadel (1) und der Positionen der Einstichstelle und des Zielpunktes (PE, PT);
- Anzeigen mittels des Grafikdisplays (6) der grafischen Navigationsinformation;
wobei das Verfahren **dadurch gekennzeichnet ist, dass** die Generierung der grafischen Navigationsinformation aufweist:
- Berechnen der Lage (*direction*) und Richtung (*sense*) eines ersten Vektors (V1), der aus dem relativen Ortsvektor besteht, in Bezug auf die aktuelle Position der Nadel (1) in der ersten Ebene (PE), der orthogonalen Projektion der Einstichstelle (PR) auf die erste Ebene (PR);
- Berechnen eines ersten Abstandes (D1) zwischen der aktuellen Position der Spitze (1a) der Nadel (1) und der Position der Einstichstelle (PE); und
- Generieren erster grafischer Information (201, 202), die auf die Lage und Richtung des ersten Vektors (V1) und den ersten Abstand (D1) hinweist.

9. Verfahren nach Anspruch 8, wobei das Anzeigen der grafischen Navigationsinformation aufweist:
- Anzeigen der ersten grafischen Information (201, 202) wenigstens bis der erste Abstand (D1) größer oder gleich einem ersten Grenzwert (TH1) ist.

10. Verfahren nach Anspruch 8 oder 9, wobei das Generieren einer grafischen Navigationsinformation aufweist, wenn der erste Abstand (D1) kleiner als ein erster Grenzwert (TH1) ist:
- Kalkulieren des Betrages, der Lage und Richtung eines zweiten Vektors (V2), der aus dem relativen Ortsvektor besteht, in Bezug auf die aktuelle Position der Nadel (1) in der ersten Ebene (PR), des Schnittpunktes der ersten Ebene (PR) und einer sich durch die Einstichstelle (PE) und den Zielpunkt (PT) erstreckenden geraden Linie (RI); und
- Generieren zweiter grafischer Information (301 - 303), die auf den Betrag, die Lage und Richtung des zweiten Vektors (V2) hinweist.

11. Verfahren nach Anspruch 10, wobei das Anzeigen der grafischen Navigationsinformation aufweist:
- Anzeigen der zweiten grafischen Information (30 -303) wenigstens bis der Betrag des zweiten Vektors (V2) größer oder gleich einem zweiten Grenzwert (TH2) ist.

12. Verfahren nach Anspruch 10 oder 11, wobei das Generieren der grafischen Navigationsinformation aufweist, wenn der Betrag des zweiten Vektors (V2) kleiner als der zweite Grenzwert (TH2) ist:
- Berechnen eines zweiten Abstandes (D2) zwischen der aktuellen Position der Spitze (1a) der Nadel (1) und der Position des Zielpunktes (PT); und
- Generieren dritter grafischer Information (401), die auf den zweiten Abstand (D2) hinweist;
Anzeigen der grafischen Navigationsinformation aufweisend:
- Anzeigen der dritten grafischen Information bis der zweite Abstand größer oder gleich einem dritten Grenzwert (TH3) ist.

## Revendications

1. Système destiné à guider l'insertion manuelle d'une aiguille dans le corps d'un patient pendant une procédure chirurgicale percutanée, le système comprenant : un dispositif de guidage (5) qui comprend un affichage graphique (6), une pluralité de marqueurs (7a-7d) appropriés pour réfléchir la lumière infrarouge, et des moyens de support (8) pour permettre le montage du dispositif de guidage (5) sur l'aiguille (1), avec les marqueurs (7a-7d) agencés selon un certain motif géométrique par rapport à l'embout (1a) de l'aiguille (1) et avec l'écran (14) de l'affichage graphique (6) parallèle à un premier plan (PR) perpendiculaire à l'axe de l'aiguille (1) ; des moyens d'acquisition d'images infrarouges (9) pour acquérir des images du dispositif de guidage (5) pendant la procédure chirurgicale ; des moyens de mémoire (11) pour enregistrer les positions, dans un système de coordonnées qui est fixe par rapport à la table d'opération, d'un point d'entrée (PE) de l'aiguille (1) sur le corps (2) et d'un point cible (PT) à l'intérieur du corps (2) qui doit être atteint par l'embout (1a) de l'aiguille (1) ; et des moyens de traitement (10), qui sont interfacés avec lesdits moyens d'acquisition d'images (9) et avec ledit affichage graphique (6) et qui sont configurés pour déterminer en temps réel la position des marqueurs (7a-7d) dans ledit système de coordonnées sur la base des images acquises et la position actuelle de l'aiguille (1) en fonction des positions des marqueurs (7a-7d) et dudit motif géométrique, et pour commander l'affichage graphique (6) de telle sorte qu'il affiche des informations de navigation graphiques qui sont une fonction de la position actuelle de l'aiguille (1) et des positions des points d'entrée et cible (PE, PT); le système étant **caractérisé en ce que** lesdits moyens de traitement (10) sont configurés pour calculer la direction et le sens d'un premier vecteur (V1) constitué du vecteur de position relative, par rapport à la position actuelle de l'aiguille (1) sur ledit premier plan (PR), de la projection orthogonale sur ledit premier plan (PR) du point d'entrée (PE), et pour calculer une première distance (D1) entre la position actuelle de l'embout (1a) de l'aiguille (1) et la position du point d'entrée (PE) ; lesdites informations de navigation graphiques comprenant des premières informations graphiques (201, 202) indiquant ladite direction et ledit sens du premier vecteur (V1) et ladite première distance (D1).

2. Système selon la revendication 1, dans lequel ladite pluralité de marqueurs (7a-7d) comprend un premier et un deuxième marqueur (7a, 7b) alignés le long d'un premier axe (18a), qui est parallèle à un côté (14a) de l'écran (14) dudit affichage graphique (6).

3. Système selon la revendication 2, dans lequel ladite pluralité de marqueurs (7a-7d) comprend un troisième marqueur (7c) aligné avec le premier marqueur (7a) le long d'un deuxième axe (22) qui, conjointement avec ledit premier axe (18a), est situé sur un deuxième plan (P2) qui croise obliquement le premier plan (PR).

4. Système selon la revendication 3, dans lequel ladite pluralité de marqueurs (7a-7d) comprend un quatrième marqueur (7d) aligné avec le premier marqueur (7a) le long d'un troisième axe (24) qui, conjointement avec le premier axe (18a), est situé sur un troisième plan (P3) qui croise obliquement à la fois le premier et le deuxième plan (PR, P2).

5. Système selon l'une quelconque des revendications 1 à 4, dans lequel lesdits moyens de traitement (10) sont configurés pour calculer, quand ladite première distance (D1) est inférieure à un premier seuil (TH1), le module, la direction et le sens d'un deuxième vecteur (V2), qui est constitué du vecteur de position relative, par rapport à la position actuelle de l'aiguille (1) sur ledit premier plan (PR), de l'intersection entre ledit premier plan (PR) et une ligne droite (RI) passant par le point d'entrée (PE) et le point cible (PT) ; lesdites informations de navigation graphiques comprenant des deuxièmes informations graphiques (301-303) indiquant lesdits module, direction et sens du deuxième vecteur (V2).

6. Système selon la revendication 5, dans lequel lesdits moyens de traitement (10) sont configurés pour calculer, quand le module dudit deuxième vecteur (V2) est inférieur à un deuxième seuil (TH2), une deuxième distance (D2) entre la position actuelle de l'embout (1a) de l'aiguille (1) et le point cible (PT) ; lesdites informations de navigation graphiques comprenant des troisièmes informations graphiques (401) indiquant ladite deuxième distance (D2).

7. Système selon l'une quelconque des revendications 1 à 6 et comprenant des moyens d'étalonnage (26, 31, 32) ayant d'autres moyens de support (26) pour l'aiguille (1), qui présentent un premier trou (28) non débouchant pour recevoir l'embout (1a) de l'aiguille (1) et un deuxième trou (30) débouchant agencé à une certaine distance (DN) du premier trou (28) et approprié pour être traversé par la tige de l'aiguille (1) quand l'embout (1a) entre en prise avec le premier trou (28), deux groupes de quatre autres marqueurs (31, 32) chacun, qui sont appropriés pour réfléchir la lumière infrarouge et sont appliqués sur les autres moyens de support (26) de façon à définir, en ce qui concerne le premier groupe (31), les extrémités de deux premiers tronçons (27a, 27b) qui se croisent dans le premier trou (28) et, en ce qui concerne le deuxième groupe (32), les extrémités de deux deuxièmes segments (29a, 29b) qui se croisent dans le deuxième trou (30) ; lesdits moyens de traitement (10) étant configurés pour déterminer les positions de tous les marqueurs (7a-7d, 31, 32) sur la base d'au moins une image, acquise par lesdits moyens d'acquisition d'images (9), du dispositif de guidage (5) monté sur l'aiguille (1), cette dernière étant agencée dans lesdits premier et deuxième trous (28, 30), et pour déterminer ledit motif géométrique sur la base desdites positions de tous les marqueurs (7a-7d, 31, 32).

8. Procédé destiné à guider l'insertion manuelle d'une aiguille dans le corps d'un patient pendant une procédure chirurgicale percutanée, le procédé comprenant :
- l'acquisition des positions, dans un système de coordonnées qui est fixe par rapport à la table d'opération, d'un point d'entrée (PE) de l'aiguille (1) sur ledit corps (2) et d'un point cible (PT) à l'intérieur dudit corps (2) qui doit être atteint par l'embout (1a) de l'aiguille (1) ;
- le montage, sur l'aiguille (1), d'un affichage graphique (6) avec son écran (14) agencé parallèlement à un premier plan (PR), qui est perpendiculaire à l'axe de l'aiguille (1), et une pluralité de marqueurs (7a-7d) appropriés pour réfléchir le rayonnement infrarouge et agencés selon un certain motif géométrique par rapport à l'embout (1a) de l'aiguille (1) ;
- l'acquisition d'images des marqueurs (7a-7d) au-dessus de la table d'opération pendant la procédure chirurgicale au moyen de moyens d'acquisition d'images (9) ;
- la détermination en temps réel de la position, dans ledit système de coordonnées, des marqueurs (7a-7d) sur la base des images acquises et de la position actuelle de l'aiguille (1) en fonction des positions des marqueurs (7a-7d) et dudit motif géométrique,
- la génération d'informations de navigation graphiques en fonction de la position actuelle de l'aiguille (1) et des positions des points d'entrée et cible (PE, PT) ;
- l'affichage, au moyen dudit affichage graphique (6), des informations de navigation graphiques ;
le procédé étant **caractérisé en ce que** la génération d'informations de navigation graphiques comprend :
- le calcul de la direction et du sens d'un premier vecteur (V1), qui est constitué du vecteur de position relative, par rapport à la position actuelle de l'aiguille (1) sur ledit premier plan (PR), de la projection orthogonale du point d'entrée (PE) sur ledit premier plan (PR) ;
- le calcul d'une première distance (D1) entre la position actuelle de l'embout (1a) de l'aiguille (1) et la position du point d'entrée (PE) ; et
- la génération de premières informations graphiques (201, 202) qui indiquent ladite direction et ledit sens du premier vecteur (V1) et ladite première distance (D1).

9. Procédé selon la revendication 8, dans lequel l'affichage des informations de navigation graphiques comprend :
- l'affichage desdites premières informations graphiques (201, 202) au moins jusqu'à ce que la première distance (D1) soit supérieure ou égale à un premier seuil (TH1).

10. Procédé selon la revendication 8 ou 9, dans lequel la génération d'informations de navigation graphiques comprend, quand la première distance (D1) est inférieure à un premier seuil (TH1) :
- le calcul du module, de la direction et du sens d'un deuxième vecteur (V2), qui est constitué du vecteur de position relative, par rapport à la position actuelle de l'aiguille (1) sur ledit premier plan (PR), de l'intersection entre ledit premier plan (PR) et une ligne droite (RI) passant par le point d'entrée (PE) et le point cible (PT) ; et
- la génération de deuxièmes informations graphiques (301-303) qui indiquent lesdits module, direction et sens du deuxième vecteur (V2).

11. Procédé selon la revendication 10, dans lequel l'affichage des informations de navigation graphiques comprend :
- l'affichage desdites deuxièmes informations graphiques (301-303) au moins jusqu'à ce que le module du deuxième vecteur (V2) soit supérieur ou égal à un deuxième seuil (TH2).

12. Procédé selon la revendication 10 ou 11, dans lequel la génération d'informations de navigation graphiques comprend, quand le module du deuxième vecteur (V2) est inférieur au deuxième seuil (TH2) :
- le calcul d'une deuxième distance (D2) entre la position actuelle de l'embout (1a) de l'aiguille (1) et la position du point cible (PT) ; et
- la génération de troisièmes informations graphiques (401) qui indiquent la deuxième distance (D2) ;
l'affichage des informations de navigation graphiques comprenant :
- l'affichage des troisièmes informations graphiques jusqu'à ce que la deuxième distance (D2) soit supérieure ou égale à un troisième seuil (TH3).
